# EUROPEAN PATENT APPLICATION

(11) **EP 4 417 197 A1**
(43) Date of publication of application: **21.08.2024**
(21) Application number: 22881197.2
(22) Date of filing: 26.08.2022
(51) Int. Cl.: A61K 9/51, A61K 47/24, A61K 47/14, A61K 9/107, A61K 31/366, A61P 35/00, A61K 9/00

(54) **PHARMACEUTICAL COMPOSITION FOR TREATMENT OF CASTRATION-RESISTANT PROSTATE CANCER COMPRISING BRUCEANTIN AND NANOPARTICLES**

(30) Priority: 15.10.2021 KR 20210137432
(71) Applicant: Samsung Life Public Welfare Foundation, Seoul 04384 (KR); Research & Business Foundation SungKyunKwan University, Gyeonggi-do 16419 (KR)
(72) Inventor: KIM, Jeong Hoon, Seoul 05738 (KR); JACKMAN, Joshua Alexander, Suwon-si Gyeonggi-do 16420 (KR); JEONG, Byong Chang, Seoul 06305 (KR); MOON, Sue Jin, Seoul 04734 (KR); YOON, Bo Kyeong, Gwangju 62062 (KR); JEON, Won Yong, Siheung-si Gyeonggi-do 15121 (KR)
(74) Representative: Weickmann & Weickmann PartmbB
(86) International application number: PCT/KR2022/012796
(87) International publication number: WO 2023/063567

(57) **Abstract**

The present invention relates to a pharmaceutical composition for the treatment of castration-resistant prostate cancer, comprising bruceantin and nanoparticles. Using the composition comprising bruceantin and nanoparticles according to an embodiment, it is possible to effectively treat castration-resistant prostate cancer by reducing the side effects of bruceantin and overcoming the poor solubility thereof.

## Description

### Technical Field

The present invention relates to a pharmaceutical composition for treating castration-resistant prostate cancer comprising bruceantin and nanoparticles.

### Background Art

Prostate cancer is the most common cancer in men, ranking second in cancer mortality among men in the United States, and is showing a 10.5% increase every year in Korea. Thus, the importance of diagnosis and treatment of prostate cancer is emerging.

Androgen receptor (AR) is a transcription factor that plays an important role in the onset and progression of prostate cancer, and it is known that more than 90% of prostate cancer is AR-positive prostate cancer.

AR is a ligand-dependent transcription factor that is activated by binding to its ligand androgen, and prostate-specific antigen (PSA), a representative target gene of AR, is used as a representative diagnostic marker of prostate cancer.

Treatment of prostate cancer is performed using androgen deprivation therapy (hereinafter referred to as ADT), which indirectly inhibits the transcriptional activity of AR by inhibiting the synthesis of androgens, and anti-androgen therapy which inhibits the transcriptional activity of AR by direct binding to AR.

Both therapies exhibit therapeutic effects in the early stages of treatment, but prostate cancer eventually progresses to castration-resistant prostate cancer (CRPC), which is resistant to anticancer drugs. Currently, there is virtually no therapeutic method for the CRPC stage.

Various causes including AR and coactivator gene amplification, mutations, etc. are known to be responsible for the mechanisms of CRPC progression. However, in recent years, expression of AR-V7, an AR variant lacking the C-terminal ligand binding domain of the AR protein, has been reported to be the main cause of CRPC progression.

Lacking the ligand binding domain, AR-V7 may have ligand-independent transcriptional activity, and also has resistance to existing drugs developed to target the C terminus of AR, and thus acts as an important factor that promotes the progression of CRPC.

Therefore, in order to overcome the limitations of existing therapies, there is a need to develop a new therapeutic agent that simultaneously targets AR and AR-V7.

Application studies using nanoparticles in medicine have been actively conducted. These studies have been conducted in various fields, including oral formulations for improved absorption, transdermal formulations for high permeability absorption, injectable formulations for targeting or improving stability in aqueous solutions, and formulations for lung or mucous membrane penetration.

Many drugs currently used as pharmaceuticals are poorly soluble and show low bioavailability due to their low solubility when administered *in vivo.* Many of the drug candidates under development also face difficulties in formulation due to their poor solubility. Thus, many different formulation methods have been studied for solubilizing poorly soluble drugs, but to date, their effect has been minimal or their application has been limited.

Polymeric nanoparticles are one of the important fields in drug delivery systems, and recently, many studies have been conducted on the production of nanoparticles using amphiphilic polymers. Amphiphilic polymers in which hydrophobic blocks and hydrophilic blocks coexist form nano-aggregates having unique structures by physical cohesive forces such as intermolecular hydrophobic interactions and van der Waals forces in aqueous solutions. This means that the hydrophobic blocks tend to aggregate themselves to minimize their contact with water. This hydrophobic aggregate forms a core, and hydrophilic blocks surround the outside of the core, thus forming a polymer micelle having increased solubility in aqueous solutions. These polymer micelles have a core with a size of tens to hundreds of nanometers and encapsulate poorly soluble drugs therein, and thus they are widely used as a formulation for solubilizing poorly soluble drugs having low water solubility and low *in vivo* absorption rate.

Many efforts have been made to deliver anticancer drugs for cancers that are difficult to cure, based on the above-described nano-based technology, but no clear results have yet been shown. Accordingly, studies need to be conducted to increase the *in vivo* absorption rate of bruceantin, which has been reported to have a therapeutic effect by simultaneously targeting AR and AR-V7 in castration-resistant prostate cancer (Korean Patent Application Publication No. 10-2020-0139625).

### DISCLOSURE

### Technical Problem

An object of the present invention is to provide a pharmaceutical composition for treating castration-resistant prostate cancer comprising bruceantin and nanoparticles, and a method for treating castration-resistant prostate cancer.

### Technical Solution

One aspect of the present invention provides a pharmaceutical composition for treating castration-resistant prostate cancer comprising bruceantin (BCT) and nanoparticles.

According to one embodiment of the present invention, the bruceantin may be a compound having a structure of the following Formula 1:

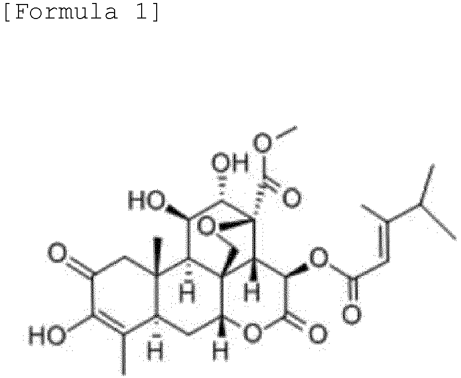

The bruceantin is known to simultaneously and strongly inhibit the transcriptional activity of AR/AR-V7 in both castration-resistant prostate cancer cell line 22RV1 cells and androgen-dependent LNCaP cells (Korean Patent Application Publication No. 10-2020-0139625).

As used in the present specification, the term "castration-resistant prostate cancer (CRPC)" refers to prostate cancer that continues to grow even when the amount of testosterone in the blood is reduced to a very low level. Early-stage prostate cancer requires androgen for growth, but castration-resistant prostate cancer, which is resistant to androgen deprivation therapy, continues to grow without androgen. Such patients have a very poor prognosis because cancer progresses even when the amount of testosterone is reduced to the level of castration using radiation, surgery, drugs, etc.

As used in the present specification, the term "treatment" refers to any action that alleviates or beneficially alters the symptoms of castration-resistant prostate cancer by administration of the pharmaceutical composition according to the present invention.

For administration, the pharmaceutical composition may preferably be formulated as a pharmaceutical composition further comprising one or more pharmaceutically acceptable carriers in addition to the pharmaceutical composition for treating castration-resistant prostate cancer comprising bruceantin and nanoparticles.

The dosage form of the pharmaceutical composition may be granules, powders, tablets, coated tablets, capsules, suppositories, solutions, syrups, juices, suspensions, emulsions, drops, or injectable solutions, or the like. For example, for the formulation into a tablet or capsule, the active ingredient may be combined with an oral, non-toxic, pharmaceutically acceptable inert carrier, such as ethanol, glycerol, or water. If desired or necessary, a suitable binder, a lubricant, a disintegrant, and a colorant may also be contained in the form of being mixed. Suitable binders include, but are not limited to, natural sugars, such as starch, gelatin, glucose, or beta-lactose, corn sweeteners, natural and synthetic gums, such as gum acacia, tragacanth, or sodium oleate, sodium stearate, magnesium stearate, sodium benzoate, sodium acetate, sodium chloride, and the like. Suitable disintegrants include, but are not limited to, starch, methyl cellulose, agar, bentonite, xanthan gum, etc.

Examples of a pharmaceutically acceptable carrier that may be used in the composition formulated into a liquid solution include saline, sterile water, Ringer's solution, buffered saline, an albumin injection solution, a dextrose solution, a maltodextrin solution, glycerol, ethanol, and a mixture of one or more thereof, which are sterilizable and biocompatible. If necessary, other ordinary additives, such as an antioxidant, a buffer solution, and a bacteriostatic agent, may be added. In addition, the composition may be formulated into an injectable formulation, such as an aqueous solution, a suspension, or an emulsion, a pill, a capsule, granules, or a tablet by further adding a diluent, a dispersant, a surfactant, a binder, and a lubricant thereto.

The appropriate dosage of the pharmaceutical composition of the present invention may be determined depending on factors such as formulation method, administration mode, patient's age, weight, sex, pathological condition, diet, administration time, administration route, excretion rate, and reaction sensitivity.

The pharmaceutical composition of the present invention may be prepared in a unit dose form or prepared to be contained in a multi-dose container by formulating with a pharmaceutically acceptable carrier and/or excipient, according to a method that may be easily carried out by a person skilled in the art to which the present invention pertains.

According to one embodiment, the nanoparticles may be self-assembled nanostructures.

As used in the present specification, the term "self-assembly" refers to an assembly method that creates nanostructures by spontaneous combination of atoms, molecules, or nanoparticles. Self-assembly is a process that runs counter to the fact that increasing disorder is a spontaneous process from a thermodynamic point of view. Thus, the self-assembly process overcomes this disorder through energy generated by interaction between particles, and sources of energy include Coulomb interaction due to charges on the particle surface, van der Waals interaction due to polarization, hydrogen bonding, interaction between hydrophilic/hydrophobic functional groups (hydrophilic/hydrophobic interaction), short-range repulsive forces, etc.

The self-assembly method is an important method for fabricating nanodevices through a bottom-up approach. This is used for the creation of functional supramolecular assemblies controlled at the molecular level, the arrangement of molecules or nanoparticles on a solid surface, and the fabrication of other nanostructures using these self-assemblies as templates.

According to one embodiment, the self-assembled nanostructures may have cell membrane-mimicking nanostructures that self-assemble from a mixture of long-chain and short-chain lipidic components.

The exact morphology of these self-assembled nanostructures may be determined by factors such as the molar ratio of long-chain to short-chain lipidic components, lipid composition, and temperature.

According to one embodiment, the long-chain lipidic component may be selected from the group consisting of dioleoyl phosphatidylethanolamine (DOPE), 1,2-diphytanoyl-sn-glycero-3-phosphoethanolamine (DPhPE), 1,2-distearoyl-sn-glycero-3-phosphoethanolamine (DSPE), 1,2-dipalmitoyl-sn-glycero3-phosphoethanolamine (DPPE), and 1,2-dioleoyl-sn-glycero-3-phosphocholine (DOPC), and preferably may be DOPC.

According to one embodiment, the short-chain lipidic component may be glycerol monolaurate (GML).

According to one embodiment, the bruceantin may be encapsulated in the nanoparticles. The bruceantin may be encapsulated in the amphiphilic shell and/or water-soluble interior of the nanoparticle, which can lower the drug concentration outside the nanoparticle and facilitate drug delivery. The bruceantin may be dispersed within the nanoparticles.

According to one embodiment, the size of the nanoparticles may be 300 to 400 nm, without being limited thereto.

The pharmaceutical composition of the present invention may be administered orally or parenterally. For parenteral administration, the pharmaceutical composition may be administered by intravenous injection, subcutaneous injection, intramuscular injection, intraperitoneal injection, transdermal administration, or the like. For oral administration, the composition may be prepared and administered in oral dosage forms. The oral dosage forms include troches, lozenges, tablets, aqueous suspensions, oily suspensions, prepared powders, granules, pills, powders, emulsions, hard capsules, soft capsules, syrups, or elixirs. The composition may be formulated in oral dosage forms using binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol wax, etc. In addition, sweeteners, fragrances, syrups, etc. may also be used. Furthermore, in the case of capsules, in addition to the above-mentioned substances, a liquid carrier such as fatty oil may be additionally used.

Another aspect of the present invention provides a method for treating castration-resistant prostate cancer, comprising a step of administering to a castration-resistant prostate cancer patient a pharmaceutical composition comprising bruceantin represented by the following Formula 1 and nanoparticles:

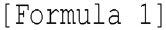

Still another aspect provides the use of bruceantin represented by the following Formula 1 and nanoparticles for treating castration-resistant prostate cancer:

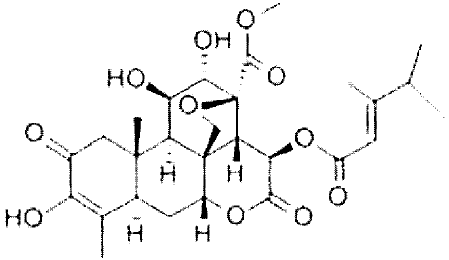

Yet another aspect provides the use of bruceantin represented by the following Formula 1 and nanoparticles for preparing a pharmaceutical composition for treating castration-resistant prostate cancer.

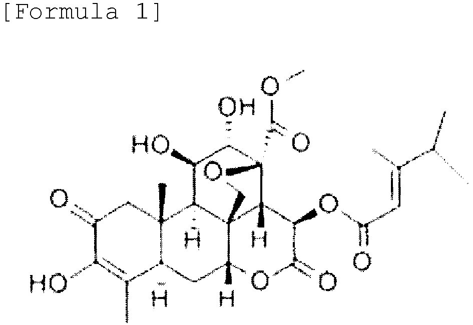

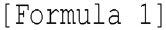

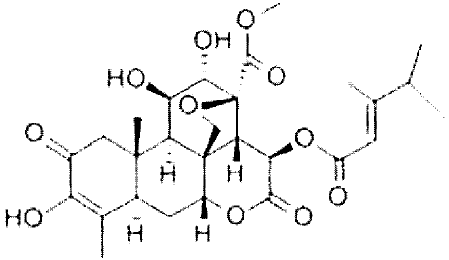

### Advantageous Effects

Using the pharmaceutical composition comprising bruceantin and nanoparticles according to one embodiment, it is possible to effectively treat castration-resistant prostate cancer by reducing the side effects of bruceantin and overcoming the poor solubility thereof.

### Brief Description of Drawings

FIG. 1 shows an optimized method for fabricating nano-bruceantin structures.
FIG. 2 shows the results of analyzing the solubility of the drug and nanoparticle size characteristics of nano-bruceantin.
FIG. 3 shows the results of evaluating the occurrence of side effects of bruceantin and nano-bruceantin upon short-term administration.
FIG. 4 shows the results of evaluating the occurrence of side effects of bruceantin and nano-bruceantin upon long-term administration.
FIG. 5 shows the results of evaluating the cell growth inhibitory effect of treatment with nano-bruceantin.
FIG. 6 shows the results of colony formation assay after treatment with nano-bruceantin.
FIG. 7 shows the results of reporter gene assay after treatment with nano-bruceantin.
FIG. 8 shows the results of immunoblotting for AR/AR-7 after treatment with nano-bruceantin.
FIG. 9 shows the results of evaluating the tumor volume after administration of nano-bruceantin to mice xenografted with castration-resistant prostate cancer cells.
FIG. 10 shows the results of measuring bioluminescence after administration of nano-bruceantin to mice xenografted with castration-resistant prostate cancer cells.

### Best Mode

One aspect provides a pharmaceutical composition for treating castration-resistant prostate cancer comprising bruceantin represented by the following Formula (1) and nanoparticles:

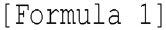

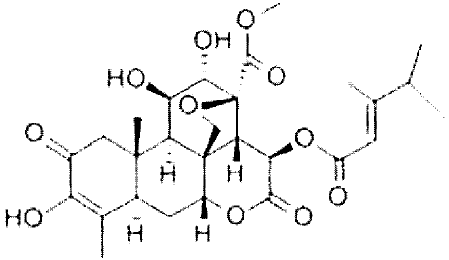

According to one embodiment, the nanoparticles may be self-assembled nanostructures.

According to one embodiment, the self-assembled nanostructures may have cell membrane-mimicking nanostructures that self-assemble from a mixture of long-chain and short-chain lipidic components.

According to one embodiment, the long-chain lipidic component may be selected from the group consisting of dioleoyl phosphatidylethanolamine (DOPE), 1,2-diphytanoyl-sn-glycero-3-phosphoethanolamine (DPhPE), 1,2-distearoyl-sn-glycero-3-phosphoethanolamine (DSPE), 1,2-dipalmitoyl-sn-glycero3-phosphoethanolamine (DPPE), and 1,2-dioleoyl-sn-glycero-3-phosphocholine (DOPC).

According to one embodiment, the short-chain lipidic component may be glycerol monolaurate.

According to one embodiment, the bruceantin may be encapsulated in the nanoparticles.

According to one embodiment, the size of the nanoparticles may be 300 to 400 nm.

According to one embodiment, the composition may be in an oral dosage form.

Another aspect provides a method for treating castration-resistant prostate cancer, comprising a step of administering to a castration-resistant prostate cancer patient a pharmaceutical composition comprising bruceantin represented by the following Formula 1 and nanoparticles:

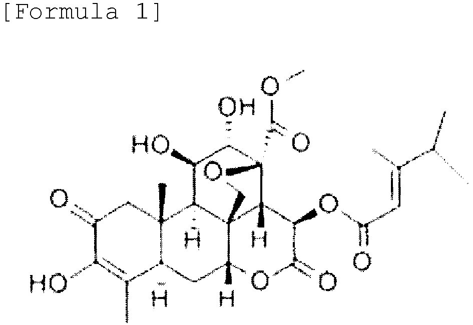

Still another aspect provides the use of bruceantin represented by the following Formula 1 and nanoparticles for treating castration-resistant prostate cancer:

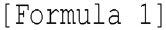

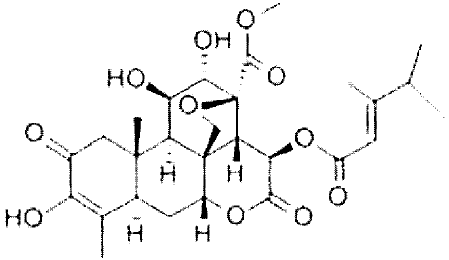

Yet another aspect provides the use of bruceantin represented by the following Formula 1 and nanoparticles for preparing a pharmaceutical composition for treating castration-resistant prostate cancer:

### Mode for Invention

One or more embodiments will be described in more detail below by way of examples. However, these examples are intended to illustrate one or more embodiments and the scope of the present invention is not limited to these examples.

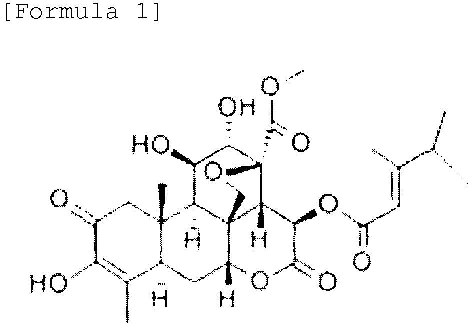

### Example 1: Examination of Whether Side Effects That Occur upon Administration of Bruceantin Alone Are Reduced upon Administration of Nano-Bruceantin

### 1-1. Mice and Material Preparation

As experimental animals, 6-week-old male nude mice (Orient Bio, Korea) were used.

1,2-dioleoyl-sn-glycero-3-phosphocholine (DOPC) was purchased from Avanti Polar Lipids, Inc. (US), and glycerol monolaurate (GML) was purchased from Abcam (UK).

### 1-2. Preparation of Nano-Bruceantin

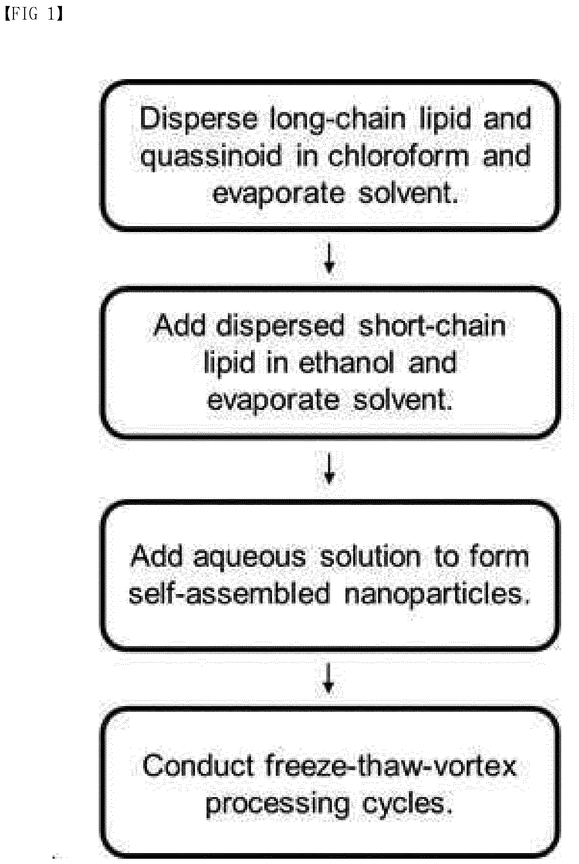

The long-chain lipidic component DOPC and the drug bruceantin were dissolved in chloroform, and then low-density nitrogen gas was injected thereinto and the chloroform solvent was removed, thus forming a dried drug-lipid film. Next, the short-chain lipidic component GML was dissolved in ethanol, and then added to the dried drug-lipid film, and the ethanol solvent was removed in the same manner using nitrogen gas. The drug-lipid film was hydrated in PBS (pH 7.4), and then the hydrated sample was processed for 5 cycles, each consisting of freezing in liquid nitrogen for 1 minute - thawing in a water bath at 60°C for 5 minutes) - strong vortexing for 30 seconds, thereby ultimately forming nanoparticles encapsulating bruceantin (FIG. 1). The final nanoparticle composition was composed of 3 mM DOPC, 12 mM GML, and 1 mg/mL nano-bruceantin 1 (nano-BCT#1), or 1 mM DOPC, 4 mM GML, and 1 mg/mL nano-bruceantin 2 (nano-BCT#2).

### 1-3. Analysis of Solubility and Size of Nano-Bruceantin

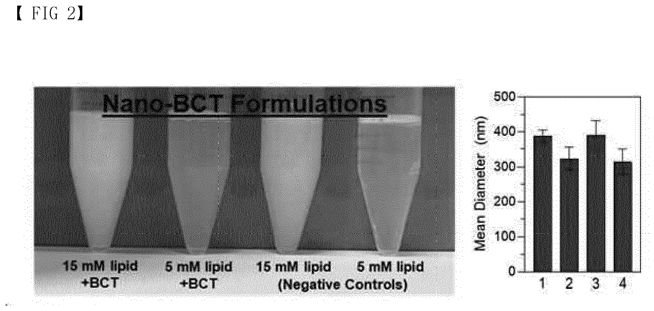

Referring to FIG. 2, as a result of visually examining the solubility of nano-bruceantin, it was confirmed that there was no agglomerated powder, indicating that the solubility of the drug increased. As a result of measuring the size of nano-bruceantin by dynamic light scattering (DLS) (also, measuring the size of a non-drug-loaded nanoparticle sample for comparison), it was confirmed that the average diameter of nano-bruceantin was about 300 to 400 nm. The results of UV-Vis spectroscopy showed that, while bruceantin alone was substantially insoluble in an aqueous solution (<0.1 mg/mL), the solubility of nano-bruceantin in an aqueous solution was at least 1 mg bruceantin/mL, indicating that the solubility of bruceantin of nano-bruceantin in an aqueous solution was improved.

Various combinations of long-chain phospholipids, medium-chain fatty acids, and monoglycerides were tested at various q-ratios and lipid-to-bruceantin ratios conditions to measure colloidal stability and release profiles, and the final composition and preparation method of bruceantin-nanoparticles were optimized.

### 1-4. Evaluation of Short-Term Toxicity of Bruceantin and Nano-Bruceantin

### 1-4-1. Experimental Method

Mice were divided into a nanoparticle-administered group (nano-Veh), a phosphate buffer saline-administered group (PBS), a nano-bruceantin-administered group (nano-BCT), and a bruceantin-administered group (free-BCT). The nano-BCT group was orally administered nano-bruceantin at 5 mg/kg daily for 7 days, and the free-BCT group was orally administered bruceantin at 5 mg/kg daily for 7 days.

### 1-4-2. Experimental Results

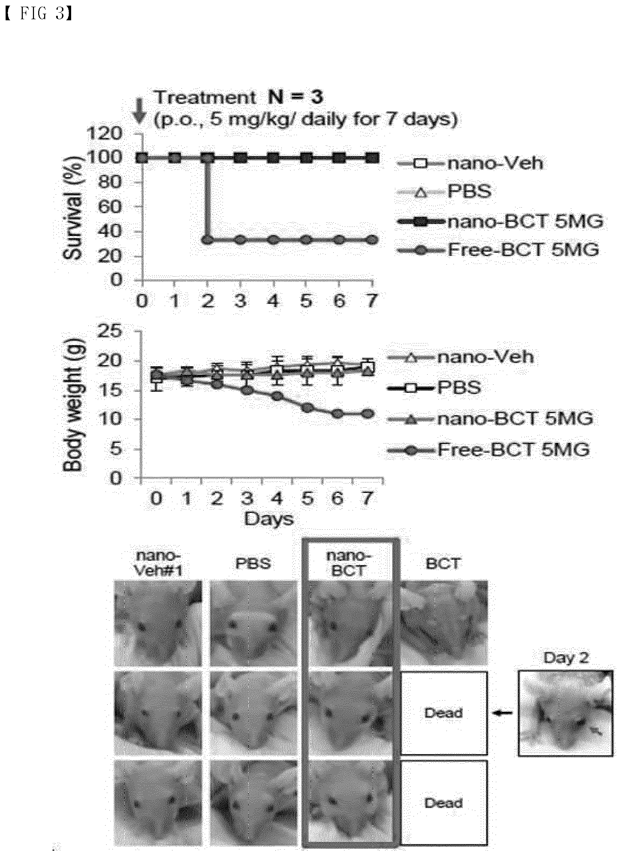

It was shown that, in the free-BCT group, short-term oral administration of bruceantin was accompanied by acute side effects such as exophthalmos, sudden death, and weight loss, and in the nano-BCT group, these side effects were not observed (FIG. 3).

### 1-5. Results of Long-Term Toxicity Evaluation of Bruceantin

### 1-5-1. Experimental Method

The nano-BCT group was orally administered nano-bruceantin at 2 mg/kg every two days for 17 days, and the free-BCT group was orally administered bruceantin at 2 mg/kg every two days for 17 days.

### 1-5-2. Experimental Results

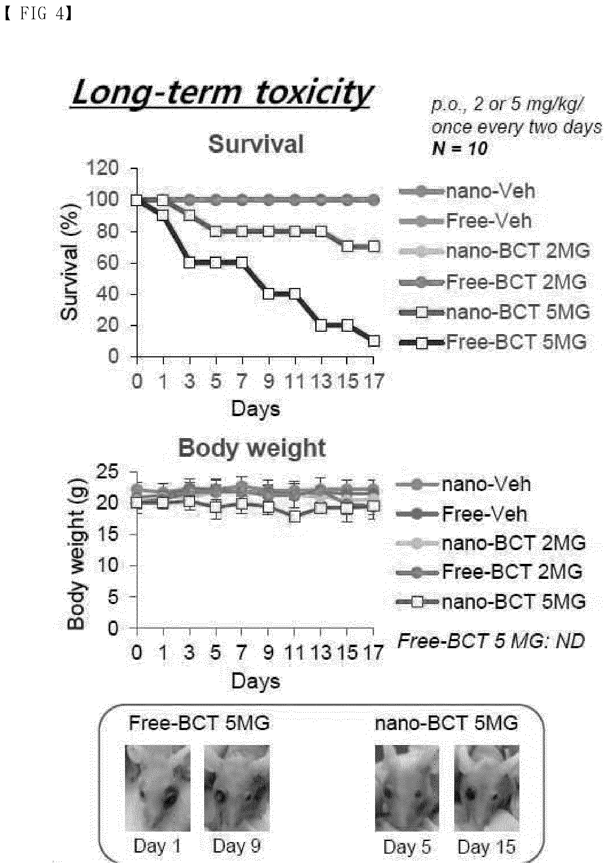

It was shown that, in the free-BCT group, long-term oral administration of bruceantin was accompanied by side effects such as exophthalmos, sudden death, and weight loss, and in the nano-BCT group, these side effects were alleviated (FIG. 4).

### Example 2: Evaluation of Therapeutic Effect of Nano-Bruceantin against Castration-Resistant Prostate Cancer

### 2-1. In Vitro Experiment

### 2-1-1. Experimental Method

Castration-resistant prostate cancer cell line 22RV1 cells were cultured with RPMI-1640 (Welgene) medium containing 10% fetal bovine serum (FBS) (Welgene) and 1% penicillin/streptomycin.

Castration-resistant prostate cancer cell line 22RV1 cells were treated with various concentrations of each of bruceantin (BCT), nano-bruceantin 1 (nano-BCT#1), and nano-bruceantin 2 (nano-BCT#2) and subjected to cell growth inhibition assay and colony formation assay. Reporter gene assay was performed by transfecting 22RV1 cells with an MMTV-LUC reporter, which can measure the transcriptional activities of AR and AR-V7, and treating the cells with various concentrations of each of BCT, nano-BCT#1, and nano-BCT#2 depending on the presence or absence of the androgen dihydrotestosterone (DHT) (DHT+, AR transcriptional activity measurement condition; DHT-, AR-V7 transcriptional activity measurement condition). Immunoblotting was performed by treating 22RV1 cells with various concentrations of each of BCT and nano-BCT, extracting protein from the cells, electrophoresing the protein on 4 to 12% SDS-PAGE gel, and transferring the electrophoresed protein to a PVDF membrane, followed by incubation with antibody.

### 2-1-2. Experimental Results

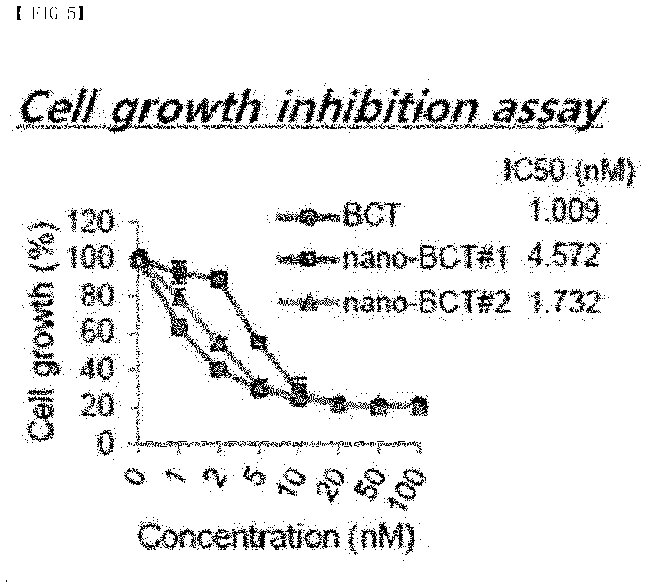

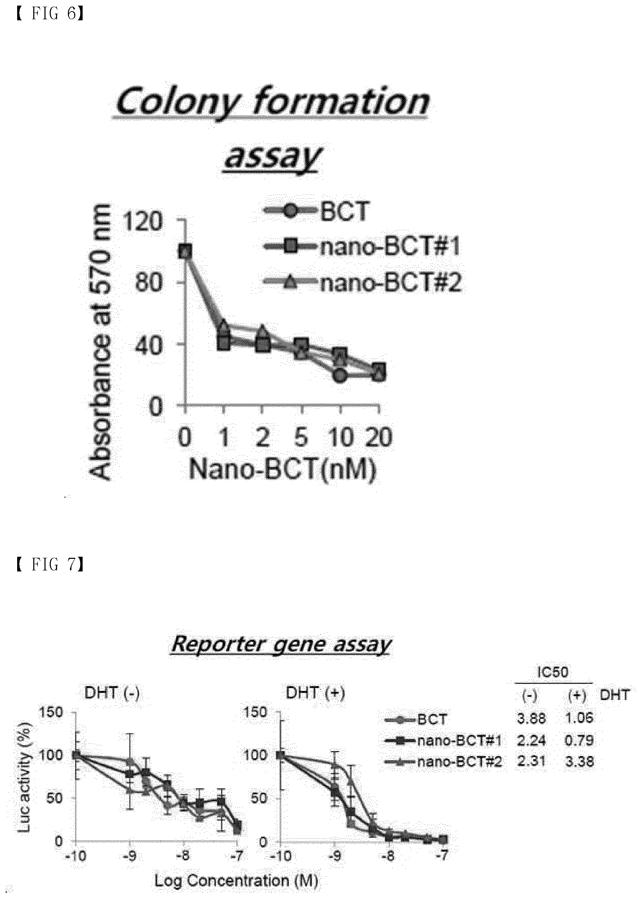

In the cell growth inhibition assay, the cell growth inhibitory effects in the BCT group, the nano-BCT#1 group, and the nano-BCT#2 group at a concentration of 10 nM or more were similar (FIG. 5). In the colony formation assay, it was shown that there was no significant difference between the results of the BCT group, the nano-BCT#1 group, and the nano-BCT#2 group (FIG. 6).

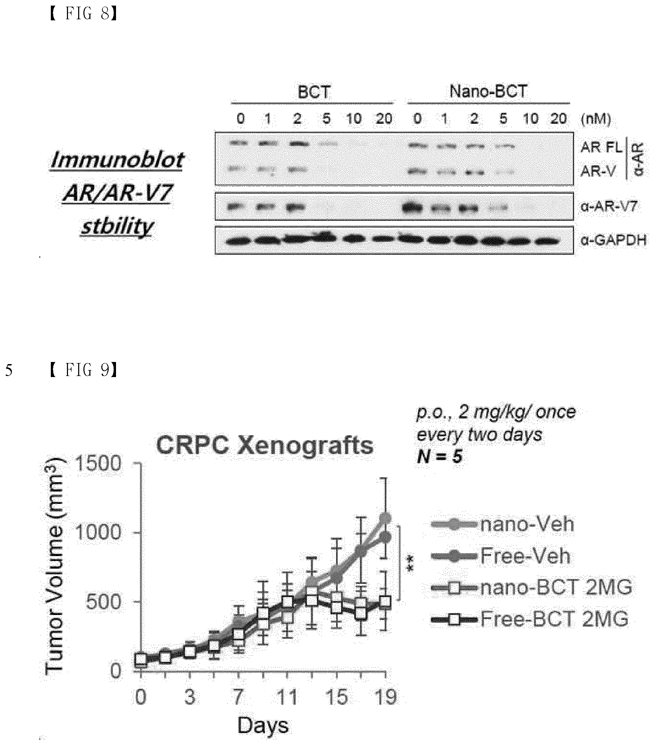

In addition, the reporter gene assay showed that there was no significant difference between the results of the BCT group, the nano-BCT#1 group, and the nano-BCT#2 group (FIG. 7). The immunoblotting results showed that AR and AR-V7 were decreased in all of the BCT group, the nano-BCT#1 group and the nano-BCT#2 group (FIG. 8).

### 2-2. In Vivo Experiment

### 2-2-1. Experimental Method

Castration-resistant prostate cancer cell line CWR22RV1 was purchased from ATCC (USA), and xenograft mice were constructed by subcutaneously injecting CWR22RV1 into nude mice.

The xenograft mice were divided into a nanoparticle-treated group (nano-Veh), a control group (free-Veh), a nano-bruceantin-treated group (nano-BCT), and a bruceantin-treated group (free-BCT). The nano-BCT group and the free-BCT group were orally administered nano-bruceantin and bruceantin, respectively, at 2 mg/kg every two days for 19 days. Thereafter, tumor volume and bioluminescence were measured for each treated group.

### 2-2-2. Experimental Results

It was shown that a decrease in tumor volume was from day 13 in the nano-BCT and free-BCT groups, excluding the nano-Veh and free-Veh groups, and there was no significant difference in the degree of decrease in tumor volume between the nano-BCT group and the free-BCT group (FIG. 9).

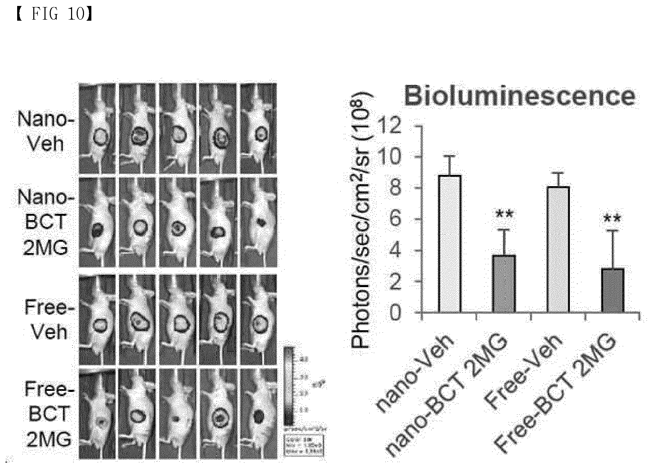

In addition, it was shown that a decrease in bioluminescence was found in the nano-BCT and free-BCT groups, excluding nano-Veh and free-Veh, and there was no significant difference in the degree of decrease in bioluminescence between the nano-BCT group and the free-BCT group (FIG. 10). Based on the above results, it was shown that nano-bruceantin did not impair the therapeutic effect of bruceantin against castration-resistant prostate cancer.

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A pharmaceutical composition for treating castration-resistant prostate cancer comprising bruceantin represented by the following Formula (1) and nanoparticles:

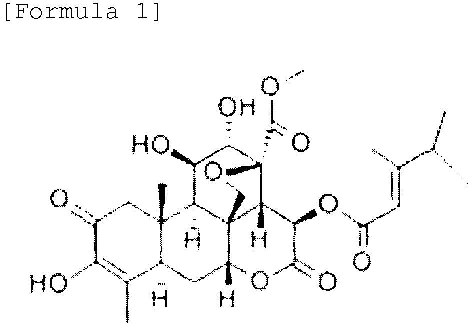

2. The pharmaceutical composition according to claim 1, wherein the nanoparticles are self-assembled nanostructures.

3. The pharmaceutical composition according to claim 2, wherein the self-assembled nanostructures have cell membrane-mimicking nanostructures that self-assemble from a mixture of long-chain and short-chain lipidic components.

4. The pharmaceutical composition according to claim 3, wherein the long-chain lipidic component is selected from the group consisting of dioleoyl phosphatidylethanolamine (DOPE), 1,2-diphytanoyl-sn-glycero-3-phosphoethanolamine (DPhPE), 1,2-distearoyl-sn-glycero-3-phosphoethanolamine (DSPE), 1,2-dipalmitoyl-sn-glycero3-phosphoethanolamine (DPPE), and 1,2-dioleoyl-sn-glycero-3-phosphocholine (DOPC).

5. The pharmaceutical composition according to claim 3, wherein the short-chain lipidic component is glycerol monolaurate.

6. The pharmaceutical composition according to claim 1, wherein the bruceantin is encapsulated in the nanoparticles.

7. The pharmaceutical composition according to claim 1, wherein the nanoparticles have a size of 300 to 400 nm.

8. The pharmaceutical composition according to claim 1, wherein the composition is in an oral dosage form.

9. A method for treating castration-resistant prostate cancer, comprising a step of administering to a castration-resistant prostate cancer patient a pharmaceutical composition comprising bruceantin represented by the following Formula 1 and nanoparticles:

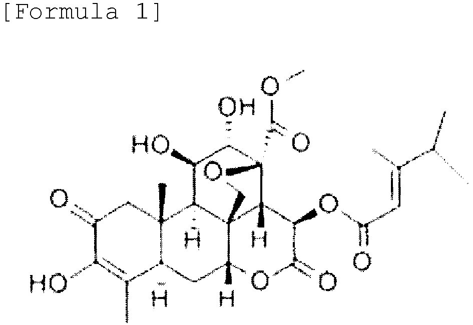

10. Use of bruceantin represented by the following Formula 1 and nanoparticles for treating castration-resistant prostate cancer:

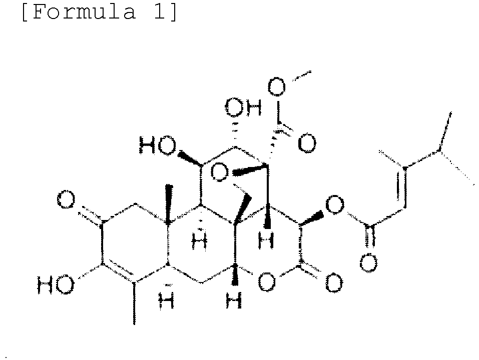

11. Use of bruceantin represented by the following Formula 1 and nanoparticles for preparing a pharmaceutical composition for treating castration-resistant prostate cancer:
